# EUROPEAN PATENT APPLICATION

(11) **EP 1 607 087 A1**
(43) Date of publication of application: **21.12.2005**
(21) Application number: 03715485.3
(22) Date of filing: 27.03.2003
(51) Int. Cl.: A61K 9/20, A61K 9/48, A61K 45/00, A61K 47/30, A61K 31/513, A61K 31/58, A61P 1/04, A61P 35/00

(54) **MEDICINAL ORAL PREPARATIONS FOR COLON DELIVERY, MEDICINAL ORAL PREPARATIONS FOR TREATING COLON CANCER AND MEDICINAL ORAL PREPARATIONS FOR TREATING COLITIS**

(71) Applicant: HISAMITSU PHARMACEUTICAL CO., INC., Tosu-shi, Saga 841-0017 (JP)
(72) Inventor: SATO, Shuji, Hisamitsu Pharmaceutical Co., Inc., Tsukuba-shi, Ibaraki 305-0856 (JP); GOTO, Takeshi, Hisamitsu Pharmaceutical Co., Inc., Tsukuba-shi, Ibaraki 305-0856 (JP); TANIDA, Norifumi, Hisamitsu Pharmaceutical Co. Inc, Tsukuba-shi, Ibaraki 305-0856 (JP); MENO, Tatsuya, Hisamitsu Pharmaceutical Co., Inc., Tsukuba-shi, Ibaraki 305-0856 (JP); YOSHINAGA, Takaaki, Hisamitsu Pharmaceut. Co., Inc, Tsukuba-shi, Ibaraki 305-0856 (JP); YONEMURA, Keishi, Hisamitsu Pharmaceutical Co. Inc, Tsukuba-shi, Ibaraki 305-0856 (JP)
(74) Representative: Forstmeyer, Dietmar
(86) International application number: PCT/JP2003/003804
(87) International publication number: WO 2004/087109

(57) **Abstract**

A medicinal oral preparation to be delivered to the large intestine comprising a core containing a pharmacologically active ingredient, an inner layer containing one or more cationic polymers and an outer layer containing one or more anionic polymers whereby the core is coated, which is designed so that, in a disintegration test successively consisting of a vertical movement for 2 hours in a first solution of pH 1.2, a vertical movement for 2 hours in a second solution of pH 7.4 and a vertical movement in a third solution of pH 6.4, the average disintegration initiation point and the average disintegration completion point each falls within a period from 35 minutes to 130 minutes after starting the vertical movement in the third solution. Namely, a medicinal oral prepa ration to be delivered to the large intestine, a medicinal oral preparation for treating colon cancer and a medicinal oral preparation for treating colitis which would not disintegrate in the stomach or small intestine but begin to disintegrate after attaining the large intestine and surely complete the disintegration while remaining in the large intestine.

## Description

### TECHNICAL FIELD

The present invention relates to a medicinal oral preparation for colon delivery, a medicinal oral preparation for treating colon cancer, and a medicinal oral preparation for treating colitis, which enable a drug to be reliably delivered to the colon.

### BACKGROUND ART

In recent years, techniques for delivering a drug to the colon have been actively developed with the object of making a peptide into an oral preparation or treating a local colon disease such as colon cancer or colitis. Since conventional oral preparations usually undergo disintegration and leaching before reaching the colon, an orally administered biologically active polypeptide or oligonucleotide is susceptible to decomposition by a hydrolase within the small intestine. Furthermore, when an anti-inflammatory drug or an anticancer drug is orally administered with the purpose of treating a colon disease such as colon cancer or colitis, in a normal administration form the drug is absorbed in the small intestine before reaching the locality of the colon, which is the location of the disease.

Under such circumstances, the development of various colon delivery techniques is currently being attempted. For example, an oral preparation formed, with the colon as a release target, by combining a polymer that only dissolves at a pH of 5.5 or higher and an insoluble polymer (EP Pat. No. 49590), a solid oral administration form coated with an appropriate amount of an anionic copolymer that dissolves at a pH of 7.0 or higher (product name: Eudragit S, manufactured by Rohm Ltd.) (International Patent Application WO83/00435), an oral preparation coated with an anionic copolymer that dissolves at pH of 7.0 or higher (product name: Eudragit S, manufactured by Rohm Ltd.) and a water-insoluble methacrylic acid ester copolymer (product name: Eudragit RS, manufactured by Rohm Ltd.) at an appropriate composition ratio (EP Pat. No. 225189), an osmotic pressure pump preparation coated with an enteric coating polymer (Belgian Pat. No. 903502), a colon-reachable medicinal oral preparation formed by coating an internal layer that dissolves at a pH of 7.0 or higher with, as an intermediate layer, a gelled polymer layer, and further thereon a stomach-resistant external layer that dissolves at a pH of 5.5 or higher (published Japanese translation No. 4-501411 of a PCT application), etc. are known. Furthermore, several colon delivery techniques employing a coating polymer for a medicinal additive have been reported (International Patent Application W090/13286, JP, A, 9-87169, International Patent Application WO95/28963).

The inventors of this invention have also proposed a lower gastrointestinal-releasing medicinal oral preparation that has high specificity for the colon (International Patent Application W094/10983, International Patent Application WO98/05310) . This is a preparation characterized by being formed from a double covering structure in which there is a compressed tablet or a capsule enclosing granules, a powder, or a liquid agent as a core, and this is covered with an inner layer formed from a cationic polymer and an outer layer formed from an anionic polymer. This preparation has excellent specificity for the colon, and enables a drug targeted for the colon to be released reliably and quickly.

### DISCLOSURE OF INVENTION

However, in subsequent research, the present inventors have found that there are cases in which merely specifying and optimizing the type and the thickness of the polymer covering the core is not sufficient; the disintegration properties of the final preparation are affected by the physicochemical properties of the tablet or capsule used as the core, and this causes the reliability of colon disintegration to be lost. That is, it has been clarified that the formulation for a tablet or a capsule used as the core, in particular the type and the amount of a pharmacologically active material contained in the core, affects the disintegration properties of the preparation after being delivered to the vicinity of the colon, and merely specifying the coating film cannot reliably guarantee the disintegration performance in the human colon. For example, when a core having a long disintegration time is used for a tablet, there are cases in which disintegration of the preparation cannot be completed while it resides in the colon. On the other hand, with regard to a preparation having a short disintegration time, when the preparation stays at the end of the ileum, there are cases in which it starts disintegrating just before reaching the colon.

These cases are due to the properties of the formulated core rather than the outer covering layer; for example, when a pharmacologically active component forming a core has the property of easily attracting water, moisture within the small intestine is taken into the core through the coated film, and although the inner layer does not dissolve since it is formed from a cationic polymer that does not dissolve in the small intestine, it cannot withstand the increase in pressure within the core and will burst. When such a preparation is actually used on humans, if the residence time within the small intestine is extended due to individual differences, etc., it will disintegrate in the small intestine.

On the other hand, if a pharmacologically active component forming a core has strong water repellency or associative strength, the disintegration time of the core naturally becomes long. When such a preparation is administered to humans, it reaches the colon, and even if the inner layer formed from a cationic polymer that dissolves in the colon dissolves immediately, since the core has strong water repellency or associative strength, the core does not disintegrate at all, and not only can the drug not be expected to be released appropriately in the colon, but in the worst case it might be excreted in the feces without completing disintegration.

The small intestine is a location where gastrointestinal movement, including peristaltic movement, is active in order to digest and absorb the contents. The administered preparation continues to receive considerable pressure within the small intestine when passing through the small intestine. If the formulation of the core has a degree of hardness that cannot withstand the pressure or friction applied within the gastrointestinal tract, it bursts in the small intestine. When such a preparation is actually used on humans, if the residence time within the small intestine is extended due to individual differences, etc. it will disintegrate in the small intestine.

Therefore, the actual disintegration properties of a preparation in the human body are affected by the properties of the core, that is, the moisture attracting properties, water repellency, and associative strength of the pharmacologically active component forming the core, the friction resistance and pressure resistance of the core, etc., and it has been found that by merely specifying the type and amount of a polymer covering the core, a drug cannot always be delivered reliably to the colon.

That is, while taking into consideration the results of the above-mentioned investigation, the object of the present invention is to solve the conventional problems and provide a medicinal oral preparation for colon delivery, a medicinal oral preparation for treating colon cancer and a medicinal oral preparation for treating colitis which will not disintegrate in the stomach or small intestine but will begin to disintegrate after reaching the colon and reliably complete disintegration while remaining in the colon.

While attempting to prepare various preparations for colon delivery having different physicochemical properties by changing the formulation of the core of the conventional preparation based on the above-mentioned knowledge obtained by the present inventors with respect to the conventional medicinal oral preparation for colon delivery comprising two polymer coating layers covering a core, the present inventors have found preparation disintegration test conditions for specifying a preparation that reliably guarantee the human colon disintegration performance and physical properties that the preparation should have under the test conditions, and have also found that this can easily solve the above-mentioned problems, and the present invention has thus been accomplished.

That is, the present invention relates to a medicinal oral preparation for colon delivery comprising a core containing a pharmacologically active component and, covering the core, an inner layer containing one or more cationic polymers and an outer layer containing one or more anionic polymers, the preparation being designed so that, in a disintegration test comprising vertical movement for 2 hours in a first solution of pH 1.2, subsequent vertical movement for 2 hours in a second solution of pH 7.4, and final vertical movement in a third solution of pH 6.4, the average disintegration initiation time and the average disintegration completion time each fall within a period from 35 min to 130 min after starting the vertical movement in the third solution.

Furthermore, the present invention relates to the medicinal oral preparation for colon delivery, wherein the preparation is designed so that the average disintegration initiation time is 35 min to 115 min and the average disintegration completion time is 50 min to 130 min after starting the vertical movement in the third solution.

Moreover, the present invention relates to the medicinal oral preparation for colon delivery, wherein the core is a solid preparation or a capsule.

Furthermore, the present invention relates to the medicinal oral preparation for colon delivery, wherein the cationic polymer dissolves or swells at a pH of 6.5 or lower, and the anionic polymer dissolves at a pH of 6.5 or higher.

Moreover, the present invention relates to the medicinal oral preparation for colon delivery, wherein the cationic polymer is one selected from the group consisting of a copolymer of methyl methacrylate, butyl methacrylate, and dimethylaminoethyl methacrylate (aminoalkyl methacrylate copolymer) and polyvinyl acetal diethylaminoacetate, and the weight of the inner layer relative to the core is 5 to 15 wt %.

Furthermore, the present invention relates to the medicinal oral preparation for colon delivery, wherein the anionic polymer is one selected from the group consisting of a copolymer of methacrylic acid and methyl methacrylate, hydroxypropylmethyl cellulose phthalate, hydroxypropylmethyl cellulose acetate succinate, carboxymethylethyl cellulose, cellulose acetate phthalate, and cellulose acetate succinate, and the weight of the outer layer relative to the core is 5 to 15 wt %.

Moreover, the present invention relates to the medicinal oral preparation for colon delivery, wherein the core contains one or more types selected from the group consisting of a disintegrating agent, a pH adjusting agent, a thickening agent, a binder, and a saccharide.

Furthermore, the present invention relates to the medicinal oral preparation for colon delivery, wherein the core contains as the disintegrating agent 3 to 15 wt % of one or more types selected from the group consisting of crospovidone, pregelatinized starch, sodium carboxymethyl starch, carmellose, calcium carmellose, sodium carmellose, powdered agar, sodium croscarmellose, low-substituted hydroxypropyl cellulose, starch, dextrin, hydroxyethylmethyl cellulose, carboxymethyl cellulose, hydroxypropyl starch, Macrogol, and mannitol.

Moreover, the present invention relates to the medicinal oral preparation for colon delivery, wherein the core contains as the pH adjusting agent 5 to 20 wt % of a weakly acidic amino acid comprising one or more types selected from the group consisting of phenylalanine, alanine, aspartic acid, glutamine, glutamic acid, methionine, glycine, and cysteine.

Furthermore, the present invention relates to the medicinal oral preparation for colon delivery, wherein the core contains as the pH adjusting agent 5 to 20 wt % of a basic amino acid comprising one or more types selected from the group consisting of arginine, lysine, and histidine.

Moreover, the present invention relates to the medicinal oral preparation for colon delivery, wherein the core contains as the pH adjusting agent 0.1 to 3 wt % of an organic acid comprising one or more types selected from the group consisting of citric acid, fumaric acid, succinic acid, and tartaric acid.

Furthermore, the present invention relates to the medicinal oral preparation for colon delivery, wherein the core contains as the thickening agent 5 to 30 wt % of one or more types selected from the group consisting of hydroxypropyl cellulose, guar gum, hydroxypropylmethyl cellulose, polyvinyl pyrrolidone, xanthan gum, and gum arabic.

Moreover, the present invention relates to the medicinal oral preparation for colon delivery, wherein the pharmacologically active component is selected from the group consisting of a peptide, a protein, an antisense drug, an anti-inflammatory drug, an antitumor drug, an antibiotic, a chemotherapeutic drug, a probiotic, an antidiarrheal drug, a purgative, and a laxative.

Furthermore, the present invention relates to the medicinal oral preparation for colon delivery, wherein the core has a diameter of 5 to 8 mm and a thickness of 3 to 6 mm.

Moreover, the present invention relates to a medicinal oral preparation for treating colon cancer comprising a core containing 10 to 70 wt % of fluorouracil and, covering the core, an inner layer containing one or more cationic polymers and an outer layer containing one or more anionic polymers.

Furthermore, the present invention relates to the medicinal oral preparation for treating colon cancer, wherein the preparation is designed so that, in a disintegration test comprising vertical movement for 2 hours in a first solution of pH 1.2, subsequent vertical movement for 2 hours in a second solution of pH 7.4, and final vertical movement in a third solution of pH 6.4, the average disintegration initiation time and the average disintegration completion time each fall within a period from 35 min to 130 min after starting the vertical movement in the third solution.

Moreover, the present invention relates to the medicinal oral preparation for treating colon cancer, wherein the preparation is designed so that the average disintegration initiation time is 35 min to 115 min and the average disintegration completion time is 50 min to 130 min after starting the vertical movement in the third solution.

Furthermore, the present invention relates to the medicinal oral preparation for treating colon cancer, wherein the core contains one or more types selected from the group consisting of a binder, a disintegrating agent, and a saccharide.

Moreover, the present invention relates to the medicinal oral preparation for treating colon cancer, wherein the core contains 5 to 40 wt % of the binder, and the mixing ratio of fluorouracil and the binder is 1:0.5 to 1:5.

Furthermore, the present invention relates to the medicinal oral preparation for treating colon cancer, wherein the binder is one or more types selected from the group consisting of crystalline cellulose, gum arabic, sodium alginate, ethyl cellulose, agar, a carboxyvinyl polymer, carmellose, gelatin, low-substituted hydroxypropyl cellulose, starch, dextrin, hydroxypropyl cellulose, hydroxypropylmethyl cellulose, pectin, polyvinyl pyrrolidone, Macrogol, and methyl cellulose.

Moreover, the present invention relates to the medicinal oral preparation for treating colon cancer, wherein the core contains 2 to 15 wt % of the disintegrating agent, and the mixing ratio of fluorouracil and the disintegrating agent is 1:0.05 to 1:1.

Furthermore, the present invention relates to the medicinal oral preparation for treating colon cancer, wherein the disintegrating agent is one or more types selected from the group consisting of crospovidone, pregelatinized starch, sodium carboxymethyl starch, carmellose, calcium carmellose, sodium carmellose, powdered agar, sodium croscarmellose, low-substituted hydroxypropyl cellulose, starch, dextrin, hydroxyethylmethyl cellulose, hydroxypropyl starch, Macrogol, and mannitol.

Moreover, the present invention relates to the medicinal oral preparation for treating colon cancer, wherein the core contains 20 to 60 wt % of the saccharide.

Furthermore, the present invention relates to the medicinal oral preparation for treating colon cancer, wherein the saccharide is one or more types selected from the group consisting of monosaccharides and disaccharides of lactose, fructose, sucrose, glucose, xylitol, maltose, mannitol, and sorbitol, polysaccharides of cellulose, hydroxypropyl cellulose, hydroxypropylmethyl cellulose, ethyl cellulose, starch, dextrin, dextran, pectin, and pullulan, and derivatives thereof.

Moreover, the present invention relates to a medicinal oral preparation for treating colitis comprising a core containing 0.5 to 10 wt % of budesonide and, covering the core, an inner layer containing one or more cationic polymers and an outer layer containing one or more anionic polymers.

Furthermore, the present invention relates to the medicinal oral preparation for treating colitis, wherein the preparation is designed so that, in a disintegration test comprising vertical movement for 2 hours in a first solution of pH 1.2, subsequent vertical movement for 2 hours in a second solution of pH 7.4, and final vertical movement in a third solution of pH 6.4, the average disintegration initiation time and the average disintegration completion time each fall within a period from 35 min to 130 min after starting the vertical movement in the third solution.

Moreover, the present invention relates to the medicinal oral preparation for treating colitis, wherein the preparation is designed so that the average disintegration initiation time is 35 min to 115 min and the average disintegration completion time is 50 min to 130 min after starting the vertical movement in the third solution.

Furthermore, the present invention relates to the medicinal oral preparation for treating colitis, wherein the core contains one or more types selected from the group consisting of a binder, a disintegrating agent, and a saccharide.

Moreover, the present invention relates to the medicinal oral preparation for treating colitis, wherein the core contains 5 to 40 wt % of the binder, and the mixing ratio of budesonide and the binder is 1:10 to 1:30.

Furthermore, the present invention relates to the medicinal oral preparation for treating colitis, wherein the binder is one or more types selected from the group consisting of crystalline cellulose, gum arabic, sodium alginate, ethyl cellulose, agar, a carboxyvinyl polymer, carmellose, gelatin, low-substituted hydroxypropyl cellulose, starch, dextrin, hydroxypropyl cellulose, hydroxypropylmethyl cellulose, pectin, polyvinyl pyrrolidone, Macrogol, and methyl cellulose.

Moreover, the present invention relates to the medicinal oral preparation for treating colitis, wherein the core contains 2 to 15 wt % of the disintegrating agent, and the mixing ratio of budesonide and the disintegrating agent is 1:2 to 1:10.

Furthermore, the present invention relates to the medicinal oral preparation for treating colitis, wherein the disintegrating agent is one or more types selected from the group consisting of crospovidone, pregelatinized starch, sodium carboxymethyl starch, carmellose, calcium carmellose, sodium carmellose, powdered agar, sodium croscarmellose, low-substituted hydroxypropyl cellulose, starch, dextrin, hydroxyethylmethyl cellulose, hydroxypropyl starch, Macrogol, and mannitol.

Moreover, the present invention relates to the medicinal oral preparation for treating colitis, wherein the disintegrating agents are crospovidone and low-substituted hydroxypropyl cellulose.

Furthermore, the present invention relates to the medicinal oral preparation for treating colitis, wherein the mixing ratio of crospovidone and low-substituted hydroxypropyl cellulose is 1:2.5 to 1:10.

Moreover, the present invention relates to the medicinal oral preparation for treating colitis, wherein the core contains 40 to 80 wt % of a saccharide.

Furthermore, the present invention relates to the medicinal oral preparation for treating colitis, wherein the saccharide is one or more types selected from the group consisting of monosaccharides and disaccharides of lactose, fructose, sucrose, glucose, xylitol, maltose, mannitol, and sorbitol, polysaccharides of cellulose, hydroxypropyl cellulose, hydroxypropylmethyl cellulose, ethyl cellulose, starch, dextrin, dextran, pectin, and pullulan, and derivatives thereof.

The vertical movement of the preparation disintegration test in the present invention is carried out using a disintegration tester in accordance with the Japanese Pharmacopoeia, United States of America Pharmacopoeia (USP), and European Pharmacopoeia (EP), and the disintegration test is carried out in accordance with these standards. Specifically, the number of times of vertical movement is 30 strokes/min. The amount of buffer solution used is adjusted so that when a tester container is at the lowest position, the upper face of the tester container coincides with the surface of the solution. The solution temperature is always maintained at 37°C while the test is being carried out.

The first solution of pH 1.2 referred to in the present invention is a solution that is adjusted to have a pH of 1.2, and a typical example thereof is a first pharmacopoeia solution of pH 1.2. The second solution of pH 7.4 is a solution that is adjusted to have a pH of 7.4, and a typical example thereof is a McIlvaine buffer solution of pH 7.4. Furthermore, the third solution of pH 6.4 is a solution that is adjusted to have a pH of 6.4, and a typical example thereof is a McIlvaine buffer solution of pH 6.4.

It has been found that the above-mentioned in vitro test conditions reflect in vivo disintegration properties in the vicinity of the colon most well, and they are advantageous in detecting small differences in the disintegration properties between preparations. For example, if the average disintegration initiation time in the above-mentioned test method is within the above-mentioned range, it can be guaranteed that the preparation can withstand residing in the end of the ileum, does not start disintegrating before entering the colon, and completely disintegrates while in a section from the ascending colon to the transverse colon, where there is an abundance of moisture. Moreover, if the average disintegration completion time in the above-mentioned test method is in the above-mentioned range, the preparation does not disintegrate at the end of the small intestine, and as expected pharmacologically active material diffuses sufficiently within the colon, in particular in a section from the ascending colon to the transverse colon. When the disintegration properties of a preparation are evaluated using this test method, if the preparation is within the above-mentioned specified time range, reliable disintegration within the colon can be guaranteed.

For example, when a preparation containing, in the core, a pharmacologically active component having moisture attracting properties is tested under the disintegration test conditions of the present invention, since moisture is taken into the core in, among the disintegration test solutions, the second solution (pH 7.4), either disintegration starts in the second solution or, even if disintegration does not start, the pressure within the core increases to some extent during incubation in the second solution, and when it is transferred to the third solution (pH 6.4), it disintegrates earlier than the disintegration time specified by the present invention. When such a preparation is actually used on humans, it might disintegrate in the small intestine if the residence time within the small intestine is extended due to individual differences, etc., and not disintegrate within the colon.

Furthermore, when a preparation containing, in the core, a pharmacologically active component having water repellency and associative strength is tested under the disintegration test conditions of the present invention, the disintegration initiation time and completion time are slower than the range specified by the present invention. Moreover, when a preparation in which the core does not have friction resistance and pressure resistance is tested under the in vitro disintegration test conditions of the present invention, since during incubation in the disintegration tester the preparation is exposed to pressure or friction accompanying vertical movement of a support plate, it cannot withstand the pressure in, among the disintegration test solutions, the second solution (pH 7.4) and starts disintegrating, or even if the disintegration does not start, since considerable mechanical pressure is applied to the preparation during vertical movement in the second solution, it results in it disintegrating earlier after it has been transferred to the third solution (pH 6.4). When such a preparation is actually used on humans, it might result in it disintegrating in the small intestine if the residence time within the small intestine is extended due to individual differences, etc.

With regard to the above-mentioned various problems, by devising the formulation of the core while taking into consideration the properties of the core, that is, the moisture attracting properties, water repellency, and associative strength of the pharmacologically active component, and the friction resistance and pressure resistance of the core, etc., it is possible to adjust the disintegration time of the preparation and easily accomplish the intended purpose.

For example, in order to reduce the disintegration time, a disintegrating agent such as crospovidone, low-substituted hydroxypropyl cellulose, or carboxymethyl cellulose, a weakly acidic amino acid such as phenylalanine, alanine, or aspartic acid, or an organic acid such as citric acid, fumaric acid, or succinic acid is added. On the other hand, in order to extend the disintegration time, a thickening agent such as hydroxypropyl cellulose, guar gum, or hydroxypropylmethyl cellulose, or a basic amino acid such as arginine, lysine, or histidine is added to the core.

In this way, by appropriately adjusting the amount, etc. of various components in the core, it is possible to easily obtain a preparation that satisfies the requirements in the above-mentioned disintegration test.

Therefore, the medicinal oral preparation for colon delivery of the present invention can ensure reliable disintegration properties in the colon as long as it has a structure comprising a core containing a pharmacologically active component and, covering the core, an inner layer containing one or more cationic polymers and an outer layer containing one or more anionic polymers, and satisfies the in vitro disintegration test conditions. That is, the preparation is not affected or changed at all by human gastric juice or in the small intestine, does not start disintegrating before reaching the colon, and can reliably complete disintegration within the colon and release the pharmacologically active component in the colon, thus enabling it to be absorbed therein. The formulation and components of the core and the core-covering layers can be freely selected from the above-mentioned adjustment means as long as the above requirements are met.

### MODES FOR CARRYING OUT THE INVENTION

Compositions and embodiments of the medicinal oral preparation for colon delivery of the present invention are explained below.

The medicinal oral preparation for colon delivery of the present invention is obtained by covering the surface of a core comprising a pharmacologically active component described below with an inner layer comprising a cationic polymer and by further covering with an outer layer comprising an anionic polymer, and satisfies the following conditions.

The medicinal oral preparation for colon delivery provided by the present invention is designed so that, when it is subjected to a disintegration test using a disintegration tester in accordance with the Japanese Pharmacopoeia, USP, or EP, in which the preparation is first subjected to a pre-treatment (vertical movement) in a first solution of pH 1.2 for 2 hours, and subsequently transferred to a second solution of pH 7.4 and subjected to a pre-treatment (vertical movement) for 2 hours, both the average disintegration initiation time and the average disintegration completion time of the preparation are between 35 min and 130 min from immediately after starting a final vertical movement in a third solution of pH 6.4, and it is preferably designed so that they are between 45 min and 127 min in order for it to reliably disintegrate in the colon. It is more preferable that the average disintegration initiation time is between 35 min and 115 min after starting vertical movement in the third solution and the average disintegration completion time is between 50 min and 130 min, and it is yet more preferable that the average disintegration initiation time is between 45 min to 110 min and the average disintegration completion time is between 60 min and 127 min.

The vertical movement in the preparation disintegration test of the present invention employs a disintegration tester in accordance with the Japanese Pharmacopoeia, United States of America Pharmacopoeia (USP), and European Pharmacopoeia (EP), and a disintegration test is carried out in accordance with these standards. Specifically, the number of times of vertical movement is 30 strokes/min. The amount of a buffer solution used is adjusted so that when a tester container is at the lowest position, the upper face of the tester container coincides with the surface of the solution. The solution temperature is always maintained at 37°C while the test is carried out.

The first solution of pH 1.2 referred to in the present invention is a solution whose pH is adjusted to 1.2, and a typical example thereof is a first pharmacopoeia solution of pH 1.2. The second solution of pH 7.4 is a solution whose pH is adjusted to 7.4, and a typical example thereof is a McIlvaine buffer solution of pH 7.4. Furthermore, the third solution of pH 6.4 is a solution whose pH is adjusted to 6.4, and a typical example thereof is a McIlvaine buffer solution of pH 6.4.

The medicinal oral preparation for colon delivery of the present invention can be prepared by mixing a pharmacologically active component with an appropriate excipient, wetting agent, disintegrating agent, fluidizing agent, pH adjusting agent, thickening agent, binder, saccharide, etc. so as to fall within the requirement ranges of the above-mentioned disintegration test, forming as a core a solid preparation in which a powder or a liquid agent is enclosed in a capsule or a powder is compression-molded, covering the surface of the core with a cationic polymer, and further covering the surface thereof with an anionic polymer, thus giving a medicinal preparation for colon delivery.

For example, in order to produce a solid preparation such as a tablet, a pharmacologically active component and additives such as a binder, an excipient, and a disintegrating agent are mixed using a mixer such as a V-type mixer, a vertical granulator, or a mortar. Subsequently, the mixture thus obtained is further mixed with an excipient such as magnesium stearate, and tableted using an appropriate tableting machine. The surface of the uncoated tablet thus obtained is then coated with a cationic polymer, and the surface thereof is further coated with an anionic polymer. Coating is carried out by continuously spraying a coating solution in a state in which the core is maintained at 30°C to 50°C. The increase in weight from an inner layer comprising a cationic polymer and an outer layer comprising an anionic polymer is preferably 5 to 15 wt % relative to the core, and more preferably 6 to 8 wt %.

With regard to the cationic polymer used in the inner layer, in order for it to dissolve in the colon, one having the property of dissolving or swelling at pH 6.5 or lower can be cited, and examples of useful polymers include, as general names, an aminoalkyl methacrylate copolymer [copolymer comprising methyl methacrylate, butyl methacrylate, and dimethylaminoethyl methacrylate, product name: Eudragit E, manufactured by Rohm Ltd.] and polyvinyl acetal diethylaminoacetate (product name: AEA, manufactured by Sankyo Co., Ltd.).

The inner layer comprising a cationic polymer is used so that, for example, it has a film thickness of 10 to 300 µm and is contained in the medicinal preparation at 1 to 40 wt %, and it is adjusted so that, when conditions of a pH of 6.0 or lower continue, the active component is quickly released from the medicinal preparation. This inner layer preferably employs an appropriate plasticizer so as to give a smooth coating film. Examples of the plasticizer include triacetin, a citric acid ester, and polyethylene glycol.

Examples of an anti-bonding agent include talc, titanium oxide, calcium phosphate, and hydrophobic light anhydrous silicic acid.

As the anionic polymer used in the outer layer, there can be cited those that have the property of easily dissolving at a pH of 6.5 or higher so that they dissolve within the small intestine, and examples of useful polymers include, as general names, methacrylic acid copolymer L [copolymer comprising methacrylic acid and methyl methacrylate, product name: Eudragit L100, manufactured by Rohm Ltd.], methacrylic acid copolymer S [copolymer comprising methacrylic acid and methyl methacrylate, product name: Eudragit S, manufactured by Rohm Ltd.], hydroxypropylmethyl cellulose acetate succinate, and hydroxypropylmethyl cellulose phthalate. The outer layer comprising an anionic polymer is used in the medicinal preparation at, for example, 1 to 40 wt %.

The medicinal oral preparation for colon delivery of the present invention contains, in the core, a disintegrating agent, a pH adjusting agent, a thickening agent, a binder, a saccharide, etc. according to the pharmacologically active component, etc. used, and they are selected appropriately.

The disintegrating agent mainly plays a role as an adjusting agent for reducing the disintegration time of the preparation; typical examples thereof include crospovidone, pregelatinized starch, sodium carboxymethyl starch, carmellose, calcium carmellose, sodium carmellose, powdered agar, sodium croscarmellose, low-substituted hydroxypropyl cellulose, starch, dextrin, hydroxyethylmethyl cellulose, carboxymethyl cellulose, hydroxypropyl starch, Macrogol, and mannitol, and it is contained in the core at, for example, 3 to 15 wt %.

As the pH adjusting agent, amino acids can be cited, and they can be divided according to the type thereof into adjusting agents that reduce the disintegration time of the preparation and adjusting agents that extend it. For example, weakly acidic amino acids such as phenylalanine, alanine, aspartic acid, glutamine, glutamic acid, methionine, glycine, and cysteine can reduce the disintegration time, and basic amino acids such as arginine, lysine, and histidine can extend the disintegration time. These amino acids are added to the core at 5 to 20 wt %.

As other pH adjusting agents, organic acids can be cited, and they play a role as adjusting agents that mainly reduce the disintegration time of the preparation. Examples thereof include organic acids such as citric acid, fumaric acid, succinic acid, and tartaric acid, and they are added to the core at 0.1 to 3 wt %.

The thickening agent plays a role as an adjusting agent that mainly extends the disintegration time of the preparation; typical examples thereof include hydroxypropyl cellulose, guar gum, hydroxypropylmethyl cellulose, polyvinyl pyrrolidone, xanthan gum, and gum arabic, and they are added to the core at 5 to 30 wt %.

Therefore, when the disintegration time of the preparation is to be reduced, the disintegrating agent, the above-mentioned weakly acidic amino acids, the organic acids, etc. are added; on the other hand, when the disintegration time is to be extended, the thickening agent, the above-mentioned basic amino acids, etc. are appropriately selected for the formulation of the core, and the medicinal preparation for colon delivery of the present invention can thus be designed.

Examples of the binder used in the present invention include crystalline cellulose, gum arabic, sodium alginate, ethyl cellulose, agar, a carboxyvinyl polymer, carmellose, gelatin, low-substituted hydroxypropyl cellulose, starch, dextrin, hydroxypropyl cellulose, hydroxypropylmethyl cellulose, pectin, polyvinylpyrrolidone, Macrogol, and methyl cellulose.

Examples of the saccharide used in the present invention include monosaccharides and disaccharides of lactose, fructose, sucrose, glucose, xylitol, maltose, mannitol, and sorbitol, polysaccharides of cellulose, hydroxypropyl cellulose, hydroxypropylmethyl cellulose, ethyl cellulose, starch, dextrin, dextran, pectin, and pullulan, and derivatives thereof.

The core formed by appropriately mixing a pharmacologically active component and the above-mentioned additives, and enclosing the powder or liquid agent in a capsule or compression-molding the powder to give a solid preparation has a diameter of, for example, 5 to 8 mm and a thickness of 3 to 6 mm.

Here, examples of the pharmacologically active component that can be enclosed in the core include an antisense drug, a peptide or protein such as calcitonin or insulin, an anti-inflammatory drug such as prednisolone, budesonide, beclomethasone, nimesulide, or 5-aminosalicylic acid, an antineoplastic such as fluorouracil or cisplatin, an antibiotic such as streptomycin, penicillin, or tetracycline, a chemotherapeutic drug, a probiotic, an antidiarrheal drug, a purgative, and a laxative.

When fluorouracil is used as the pharmacologically active component, a medicinal oral preparation for colon delivery that can treat colon cancer can be produced, and it can be produced by adding fluorouracil to the core at 10 to 70 wt %, and covering the surface of the core with an inner layer comprising the above-mentioned cationic polymer and an outer layer comprising the above-mentioned anionic polymer.

With regard to the formulation of the core when fluorouracil is used, for example, the content of the above-mentioned binder is 5 to 40 wt % in the core, the mixing ratio of fluorouracil and the binder is 1:0.5 to 1:5, the content of the above-mentioned disintegrating agent is 2 to 15 wt % in the core, the mixing ratio of fluorouracil and the disintegrating agent is 1:0.05 to 1:1, and the content of the above-mentioned saccharide is 20 to 60 wt %, thus producing the medicinal preparation for colon delivery of the present invention.

When budesonide is used as the pharmacologically active component, a medicinal oral preparation for colon delivery that can treat colitis can be produced, and it can be produced by adding budesonide to the core at 0.5 to 10 wt %, and covering the surface of the core with an inner layer comprising the above-mentioned cationic polymer and an outer layer comprising the above-mentioned anionic polymer.

With regard to the formulation of the core when budesonide is used, for example, the content of the binder is 5 to 40 wt % in the core, the mixing ratio of fluorouracil and the binder is 1:10 to 1:30, the content of the above-mentioned disintegrating agent is 2 to 15 wt % in the core, the mixing ratio of fluorouracil and the disintegrating agent is 1:2 to 1:10, and the content of the above-mentioned saccharide is 40 to 80 wt %, thus producing the medicinal preparation for colon delivery of the present invention.

When budesonide is used, a combination of crospovidone and low-substituted hydroxypropyl cellulose is preferable as the disintegrating agent, and the mixing ratio of crospovidone and low-substituted hydroxypropyl cellulose is preferably 1:2.5 to 1:10.

The medicinal oral preparation for colon delivery of the present invention, which satisfies the requirements of the above-mentioned specified range of the disintegration test in the present invention, can guarantee the performance as a medicinal preparation for colon delivery of disintegrating completely in the colon. That is, none of the preparation administered to a human will undergo any change in the stomach or the small intestine, and it will start disintegrating after reaching the colon, and at the same time as this enable the pharmacologically active component to be quickly and uniformly diffused and released in a section from the ascending colon to the transverse colon.

### Examples

The present invention is explained below in further detail by reference to Examples, but the present invention is not limited by these Examples.

### [Examples and Comparative Examples]

### <Preparation of medicinal oral preparation for colon delivery>

A tablet containing samarium trioxide, which is a tracer for gamma scintigraphy, was produced according to the formulation below. Firstly, samarium trioxide, crystalline cellulose, lactose, low-substituted hydroxypropyl cellulose, and other additives were mixed in a V type mixer for 15 min, magnesium stearate was added thereto at the end and mixed in the V type mixer for 5 min, and a tablet having a diameter of 7 mm and a weight of 195 mg was produced using a rotary tableting machine from the mixed powder thus obtained.

**Table 1**

| Formulation No. | Example 1 | Example 2 | Example 3 | Comparative Example 1 | Comparative Example 2 |
|---|---|---|---|---|---|
| Samarium trioxide | 5.13 | 5.13 | 5.13 | 5.13 | 5.13 |
| Lactose | 68.62 | 68.87 | 61.18 | 68.36 | 53.49 |
| Crystalline cellulose | 20.00 | 20.00 | 20.00 | 20.00 | 20.00 |
| Low-substituted hydroxypropyl cellulose | 5.03 | 5.03 | 5.03 | 5.03 | 5.03 |
| Citric acid | 0.26 | - | - | 0.51 | - |
| Hydroxypropyl cellulose | - | - | 7.69 | - | 15.38 |
| Magnesium stearate | 0.97 | 0.97 | 0.97 | 0.97 | 0.97 |
| * All figures in the table are expressed as parts by weight. | | | | | |

Crystalline cellulose was used as a binder, and hydroxypropyl cellulose was used as a thickening agent.

The core thus obtained was provided with the following coating as an inner layer.

| | |
|---|---|
| Eudragit E | 7 parts by weight |
| Ethanol | 70 parts by weight |
| Water | 19.5 parts by weight |
| Talc | 3.5 parts by weight |

The inner layer was applied by continuously spraying the above solution while maintaining the core at 50°C. The increase in weight of the preparation was 14 mg. After spraying, the core was dried, and further coated with the following solution.

It was further provided with the following coating as an outer layer.

| | |
|---|---|
| Eudragit S | 7.0 parts by weight |
| Ethanol | 70.0 parts by weight |
| Water | 18.8 parts by weight |
| Talc | 3.5 parts by weight |
| Polyethylene glycol 6000 | 0.7 parts by weight |

The outer layer was applied by continuously spraying the above solution while maintaining the core at 50°C. The increase in weight of the preparation was 14 mg.

### [Experimental Example 1]

### <In vitro disintegration test of samarium-containing colon-disintegrating placebo preparation>

Samarium-containing placebo tablets prepared using the formulations of Examples 1 to 3 and Comparative Examples 1 and 2 were irradiated with a neutron beam so as to make them radioactive, and then subjected to the disintegration test of the present invention with a timing based on administering the preparation to a person.

The test was carried out using a disintegration tester in accordance with the Japanese Pharmacopoeia under the conditions below.
Test conditions: the tablets were each placed in a glass tube of a tester container and subjected to vertical movement using 900 mL of a Japanese Pharmacopoeia test solution of pH 1.2 for 120 min, the test solution was then replaced with 900 mL of a test solution of pH 7.4 (McIlvaine buffer solution), and the tablets were further subjected to vertical movement for 120 min. Subsequently, the test solution was replaced with 900 mL of a test solution of pH 6.4 (McIlvaine buffer solution), the tablets were further subjected to vertical movement, and the average disintegration initiation time and the average disintegration completion time from the time at which vertical movement in the final test solution was started were recorded. The test results are given in Table 2.

**Table 2**

| Results of disintegration test of samarium-containing colon-disintegrating placebo preparation in the present invention | | | | | |
|---|---|---|---|---|---|
| Formulation No. | Example 1 | Example 2 | Example 3 | Comparative Example 1 | Comparative Example 2 |
| Average disintegration initiation time | 47 min | 92 min | 107 min | 30 min | 120 min |
| Average disintegration completion time | 61 min | 103 min | 127 min | 45 min | 135 min |

### [Experimental Example 2]

### <Dynamic behavior of samarium-containing colon-disintegrating placebo preparation in human gastrointestinal tract>

Samarium-containing placebo tablets prepared in Examples 1 to 3 and Comparative Examples 1 and 2 were irradiated with a neutron beam so as to make them radioactive, and then administered to a person, and a test for observing dynamic behavior in the gastrointestinal tract was carried out. The dynamic behavior in the gastrointestinal tract and the colon disintegration properties of the preparations were evaluated by gamma scintigraphy.

The results of the dynamic behavior test in the gastrointestinal tract using the preparations of Examples 1 to 3 and Comparative Examples 1 and 2 are given in Table 3.

**Table 3**

| Results of dynamic behavior test of samarium-containing colon-disintegrating placebo preparation in human gastrointestinal tract | | | | | |
|---|---|---|---|---|---|
| Formulation No. | Example 1 | Example 2 | Example 3 | Comparative Example 1 | Comparative Example 2 |
| End of small intestine | | | | 5% | |
| Ascending colon | 97% | 97% | 100% | 95% | 85% |
| Transverse colon | 3% | 3% | | | 5% |
| Descending colon | | | | | 5% |
| Excreted in feces | | | | | 5% |

With regard to the preparations having the formulations of Examples 1, 2, and 3, which satisfied the requirements of the present invention with respect to the average disintegration initiation time and the average disintegration completion time, when they were orally administered they showed 100% colon disintegration. 97% of the disintegration took place in the ascending colon, and the remaining 3% took place in the transverse colon.

When the preparation of Comparative Example 1, which had an average disintegration initiation time faster than the range of the requirement of the present invention, was orally administered, it disintegrated within the colon for 95% of the test subjects, but for the remaining 5% of the test subjects, it was confirmed that the preparation started disintegrating in the end of the small intestine.

With regard to the preparation of Comparative Example 2, which had an average disintegration completion time slower than the range of the requirement of the present invention, 95% showed colon disintegration, but 5% thereof did not complete disintegration before the descending colon. The remaining 5% was excreted in the feces without disintegrating.

### [Experimental Example 3]

### <In vitro disintegration test of samarium-containing colon-disintegrating placebo preparation>

Samarium-containing placebo tablets prepared using the formulations of Example 1 to 3 were irradiated with a neutron beam so as to make them radioactive, and then subjected to the disintegration test of the present invention with a timing based on administering the preparation to a person as well as to disintegration tests under the further six test conditions described below as reference examples.

The tests were carried out using a disintegration tester in accordance with the Japanese Pharmacopoeia under the conditions below.
Test conditions 1: the tablets were each placed in a glass tube of a tester container and subjected to vertical movement using 900 mL of a Japanese Pharmacopoeia test solution of pH 1.2 for 120 min, the test solution was then replaced with 900 mL of a test solution of pH 7.4 (McIlvaine buffer solution), and the tablets were further subjected to vertical movement for 120 min. Subsequently, the test solution was replaced with 900 mL of a test solution of pH 6.4 (McIlvaine buffer solution), the tablets were further subjected to vertical movement, and the disintegration initiation time and the disintegration completion time from the time at which vertical movement in the final test solution was started were recorded.
Test conditions 2: the tablets were each placed in a glass tube of a tester container and subjected to vertical movement using 900 mL of a test solution of pH 6.8 (phosphate buffer solution) for 4 hours, the test solution was then replaced with 900 mL of a test solution of pH 6.4 (phosphate buffer solution), the tablets were further subjected to vertical movement, and the disintegration initiation time and the disintegration completion time from the time at which vertical movement in the final test solution was started were recorded.
Test conditions 3: the tablets were each placed in a glass tube of a tester container and subjected to vertical movement using 900 mL of a test solution of pH 6.8 (phosphate buffer solution) for 2 hours, the test solution was then replaced with 900 mL of a test solution of pH 6.4 (phosphate buffer solution), the tablets were further subjected to vertical movement, and the disintegration initiation time and the disintegration completion time from the time at which vertical movement in the final test solution was started were recorded.
Test conditions 4: the tablets were each placed in a glass tube of a tester container and subjected to vertical movement using 900 mL of a test solution of pH 7.5 (phosphate buffer solution) for 2 hours, the test solution was then replaced with 900 mL of a test solution of pH 5.5 (acetate buffer solution), the tablets were further subjected to vertical movement, and the disintegration initiation time and the disintegration completion time from the time at which vertical movement in the final test solution was started were recorded.
Test conditions 5: the tablets were each placed in a glass tube of a tester container and subjected to vertical movement using 900 mL of a test solution of pH 6.8 (McIlvaine buffer solution) for 4 hours, the test solution was then replaced with 900 mL of a test solution of pH 6.4 (McIlvaine buffer solution), the tablets were further subjected to vertical movement, and the disintegration initiation time and the disintegration completion time from the time at which vertical movement in the final test solution was started were recorded.
Test conditions 6: the tablets were each placed in a glass tube of a tester container and subjected to vertical movement using 900 mL of a test solution of pH 6.8 (McIlvaine buffer solution) for 2 hours, the test solution was then replaced with 900 mL of a test solution of pH 6.4 (McIlvaine buffer solution), the tablets were further subjected to vertical movement, and the disintegration initiation time and the disintegration completion time from the time at which vertical movement in the final test solution was started were recorded.
Test conditions 7: the tablets were each placed in a glass tube of a tester container and subjected to vertical movement using 900 mL of a test solution of pH 7.4 (McIlvaine buffer solution) for 2 hours, the test solution was then replaced with 900 mL of a test solution of pH 5.5 (McIlvaine buffer solution), the tablets were further subjected to vertical movement, and the disintegration initiation time and the disintegration completion time from the time at which vertical movement in the final test solution was started were recorded.

The test results are given in Table 4 and Table 5.

**Table 4**

| Results of in vitro disintegration test of samarium-containing colon-disintegrating placebo preparation (average disintegration initiation time) | | | |
|---|---|---|---|
| Formulation No. | Example 1 | Example 2 | Example 3 |
| Test conditions 1 | 47 min | 92 min | 107 min |
| Test conditions 2 (reference example) | 25 min | 56 min | 98 min |
| Test conditions 3 (reference example) | 109 min | 106 min | 226 min |
| Test conditions 4 (reference example) | 5 min | 6 min | 7 min |
| Test conditions 5 (reference example) | 66 min | 118 min | 199 min |
| Test conditions 6 (reference example) | 228 min | 240 min | 381 min |
| Test conditions 7 (reference example) | 15 min | 19 min | 20 min |

**Table 5**

| Results of in vitro disintegration test of samarium-containing colon-disintegrating placebo preparation (average disintegration completion time) | | | |
|---|---|---|---|
| Formulation No. | Example 1 | Example 2 | Example 3 |
| Test conditions 1 | 61 min | 103 min | 127 min |
| Test conditions 2 (reference example) | 37 min | 66 min | 132 min |
| Test conditions 3 (reference example) | 123 min | 125 min | 273 min |
| Test conditions 4 (reference example) | 14 min | 14 min | 17 min |
| Test conditions 5 (reference example) | 79 min | 130 min | 238 min |
| Test conditions 6 (reference example) | 242 min | 254 min | 419 min |
| Test conditions 7 (reference example) | 25 min | 30 min | 33 min |

Since the above-mentioned Test conditions 2 to 7 are greatly different from the environment of the human gastrointestinal tract compared with Test conditions 1, even if the range of the disintegration time is determined under such conditions, it cannot always be said that a preparation that has been screened by such a test will disintegrate desirably in the human body. In addition, with regard to Test conditions 3, 4, and 7, the difference in time among the preparations is small, and it is difficult to distinguish the preparations. Furthermore, in Test conditions 6, the disintegration time is too long, and since a long time is required for carrying out the test, there is a practical problem. While it is easy and simple to carry out the disintegration test, Test conditions 1 closely reproduce the pH change and the environment in the body that a preparation is exposed to while passing through the stomach, the small intestine, and the colon when it is orally administered, and Test conditions 1 are therefore the most suitable

### [Example 4]

A tablet containing 5-fluorouracil was produced using the formulation below.

| | |
|---|---|
| 5-fluorouracil | 25.6 wt % |
| Lactose | 48.4 wt % |
| Crystalline cellulose | 20.0 wt % |
| L-HPC (LH-21) | 5.0 wt % |
| Magnesium stearate | 1.0 wt % |

Crystalline cellulose was used as a binder, and L-HPC (low-substituted hydroxypropyl cellulose) was used as a disintegrating agent.

The core thus obtained was provided with the following coating as an inner layer.

| | |
|---|---|
| Eudragit E | 7 parts by weight |
| Ethanol | 70 parts by weight |
| Water | 19.5 parts by weight |
| Talc | 3.5 parts by weight |

The inner layer was applied by continuously spraying the above solution while maintaining the core at 50°C. The increase in weight of the preparation was 14 mg. After spraying, the core was dried, and further coated with the following solution.

It was further provided with the following coating as an outer layer.

| | |
|---|---|
| Eudragit S | 7.0 parts by weight |
| Ethanol | 70.0 parts by weight |
| Water | 18.8 parts by weight |
| Talc | 3.5 parts by weight |
| Polyethylene glycol 6000 | 0.7 parts by weight |

The outermost layer was applied by continuously spraying the above solution while maintaining the core at 50°C. The increase in weight of the preparation was 14 mg.

A disintegration test was carried out in the same manner as in Experimental Example 1, and the average disintegration completion time was 101 min.

### [Example 5]

A tablet containing budesonide was produced using the formulation below.

| | |
|---|---|
| Budesonide | 1.0 wt % |
| Lactose | 70.8 wt % |
| Crystalline cellulose | 20.0 wt % |
| L-HPC (LH-21) | 5.1 wt % |
| Crospovidone | 1.0 wt % |
| Light anhydrous silicic acid | 1.0 wt % |
| Magnesium stearate | 1.0 wt % |

Crystalline cellulose was used as a binder, and L-HPC (low-substituted hydroxypropyl cellulose) and crospovidone were used as disintegrating agents.

The core thus obtained was provided with the following coating as an inner layer.

| | |
|---|---|
| Eudragit E | 7 parts by weight |
| Ethanol | 70 parts by weight |
| Water | 19.5 parts by weight |
| Talc | 3.5 parts by weight |

The inner layer was applied by continuously spraying the above solution while maintaining the core at 50°C. The increase in weight of the preparation was 14 mg. After spraying, the core was dried, and further coated with the following solution.

It was further provided with the following coating as an outer layer.

| | |
|---|---|
| Eudragit S | 7.0 parts by weight |
| Ethanol | 70.0 parts by weight |
| Water | 18.8 parts by weight |
| Talc | 3.5 parts by weight |
| Polyethylene glycol 6000 | 0.7 parts by weight |

The outermost layer was applied by continuously spraying the above solution while maintaining the core at 50°C. The increase in weight of the preparation was 14 mg.

A disintegration test was carried out in the same manner as in Experimental Example 1, and the average disintegration completion time was 108 min.

### Industrial Applicability

The medicinal oral preparation for colon delivery of the present invention does not undergo any change in the stomach or the small intestine, starts disintegrating reliably after reaching the colon, and at the same time can quickly release a drug. This enables preparations useful for colon disease such as colon cancer, ulcerative colitis, constipation, and diarrhea, and systemic diseases such as osteoporosis to be provided.

## Claims

1. A medicinal oral preparation for colon delivery comprising a core containing a pharmacologically active component and, covering the core, an inner layer containing one or more cationic polymers and an outer layer containing one or more anionic polymers, the preparation being designed so that, in a disintegration test comprising vertical movement for 2 hours in a first solution of pH 1.2, subsequent vertical movement for 2 hours in a second solution of pH 7.4, and final vertical movement in a third solution of pH 6.4, the average disintegration initiation time and the average disintegration completion time each fall within a period from 35 min to 130 min after starting the vertical movement in the third solution.

2. The medicinal oral preparation for colon delivery according to Claim 1, wherein the preparation is designed so that the average disintegration initiation time is 35 min to 115 min and the average disintegration completion time is 50 min to 130 min after starting the vertical movement in the third solution.

3. The medicinal oral preparation for colon delivery according to either Claim 1 or 2, wherein the core is a solid preparation or a capsule.

4. The medicinal oral preparation for colon delivery according to any one of Claims 1 to 3, wherein the cationic polymer dissolves or swells at a pH of 6.5 or lower, and the anionic polymer dissolves at a pH of 6.5 or higher.

5. The medicinal oral preparation for colon delivery according to any one of Claims 1 to 4, wherein the cationic polymer is one selected from the group consisting of a copolymer of methyl methacrylate, butyl methacrylate, and dimethylaminoethyl methacrylate (aminoalkyl methacrylate copolymer) and polyvinyl acetal diethylaminoacetate, and the weight of the inner layer relative to the core is 5 to 15 wt %.

6. The medicinal oral preparation for colon delivery according to any one of Claims 1 to 5, wherein the anionic polymer is one selected from the group consisting of a copolymer of methacrylic acid and methyl methacrylate, hydroxypropylmethyl cellulose phthalate, hydroxypropylmethyl cellulose acetate succinate, carboxymethylethyl cellulose, cellulose acetate phthalate, and cellulose acetate succinate, and the weight of the outer layer relative to the core is 5 to 15 wt %.

7. The medicinal oral preparation for colon delivery according to any one of Claims 1 to 6, wherein the core contains one or more types selected from the group consisting of a disintegrating agent, a pH adjusting agent, a thickening agent, a binder, and a saccharide.

8. The medicinal oral preparation for colon delivery according to Claim 7, wherein the core contains as the disintegrating agent 3 to 15 wt % of one or more types selected from the group consisting of crospovidone, pregelatinized starch, sodium carboxymethyl starch, carmellose, calcium carmellose, sodium carmellose, powdered agar, sodium croscarmellose, low-substituted hydroxypropyl cellulose, starch, dextrin, hydroxyethylmethyl cellulose, carboxymethyl cellulose, hydroxypropyl starch, Macrogol, and mannitol.

9. The medicinal oral preparation for colon delivery according to Claim 7, wherein the core contains as the pH adjusting agent 5 to 20 wt % of a weakly acidic amino acid comprising one or more types selected from the group consisting of phenylalanine, alanine, aspartic acid, glutamine, glutamic acid, methionine, glycine, and cysteine.

10. The medicinal oral preparation for colon delivery according to Claim 7, wherein the core contains as the pH adjusting agent 5 to 20 wt % of a basic amino acid comprising one or more types selected from the group consisting of arginine, lysine, and histidine.

11. The medicinal oral preparation for colon delivery according to Claim 7, wherein the core contains as the pH adjusting agent 0.1 to 3 wt % of an organic acid comprising one or more types selected from the group consisting of citric acid, fumaric acid, succinic acid, and tartaric acid.

12. The medicinal oral preparation for colon delivery according to Claim 7, wherein the core contains as the thickening agent 5 to 30 wt % of one or more types selected from the group consisting of hydroxypropyl cellulose, guar gum, hydroxypropylmethyl cellulose, polyvinyl pyrrolidone, xanthan gum, and gum arabic.

13. The medicinal oral preparation for colon delivery according to any one of Claims 1 to 12, wherein the pharmacologically active component is selected from the group consisting of a peptide, a protein, an antisense drug, an anti-inflammatory drug, an antitumor drug, an antibiotic, a chemotherapeutic drug, a probiotic, an antidiarrheal drug, a purgative, and a laxative.

14. The medicinal oral preparation for colon delivery according to any one of Claims 1 to 13, wherein the core has a diameter of 5 to 8 mm and a thickness of 3 to 6 mm.

15. A medicinal oral preparation for treating colon cancer comprising a core containing 10 to 70 wt % of fluorouracil and, covering the core, an inner layer containing one or more cationic polymers and an outer layer containing one or more anionic polymers.

16. The medicinal oral preparation for treating colon cancer according to Claim 15, wherein the preparation is designed so that, in a disintegration test comprising vertical movement for 2 hours in a first solution of pH 1.2, subsequent vertical movement for 2 hours in a second solution of pH 7.4, and final vertical movement in a third solution of pH 6.4, the average disintegration initiation time and the average disintegration completion time each fall within a period from 35 min to 130 min after starting the vertical movement in the third solution.

17. The medicinal oral preparation for treating colon cancer according to Claim 16, wherein the preparation is designed so that the average disintegration initiation time is 35 min to 115 min and the average disintegration completion time is 50 min to 130 min after starting the vertical movement in the third solution.

18. The medicinal oral preparation for treating colon cancer according to any one of Claims 15 to 17, wherein the core contains one or more types selected from the group consisting of a binder, a disintegrating agent, and a saccharide.

19. The medicinal oral preparation for treating colon cancer according to Claim 18, wherein the core contains 5 to 40 wt % of the binder, and the mixing ratio of fluorouracil and the binder is 1:0.5 to 1:5.

20. The medicinal oral preparation for treating colon cancer according to either Claim 18 or 19, wherein the binder is one or more types selected from the group consisting of crystalline cellulose, gum arabic, sodium alginate, ethyl cellulose, agar, a carboxyvinyl polymer, carmellose, gelatin, low-substituted hydroxypropyl cellulose, starch, dextrin, hydroxypropyl cellulose, hydroxypropylmethyl cellulose, pectin, polyvinyl pyrrolidone, Macrogol, and methyl cellulose.

21. The medicinal oral preparation for treating colon cancer according to Claim 18, wherein the core contains 2 to 15 wt % of the disintegrating agent, and the mixing ratio of fluorouracil and the disintegrating agent is 1:0.05 to 1:1.

22. The medicinal oral preparation for treating colon cancer according to either Claim 18 or 21, wherein the disintegrating agent is one or more types selected from the group consisting of crospovidone, pregelatinized starch, sodium carboxymethyl starch, carmellose, calcium carmellose, sodium carmellose, powdered agar, sodium croscarmellose, low-substituted hydroxypropyl cellulose, starch, dextrin, hydroxyethylmethyl cellulose, hydroxypropyl starch, Macrogol, and mannitol.

23. The medicinal oral preparation for treating colon cancer according to Claim 18, wherein the core contains 20 to 60 wt % of the saccharide.

24. The medicinal oral preparation for treating colon cancer according to either Claim 18 or 23, wherein the saccharide is one or more types selected from the group consisting of monosaccharides and disaccharides of lactose, fructose, sucrose, glucose, xylitol, maltose, mannitol, and sorbitol, polysaccharides of cellulose, hydroxypropyl cellulose, hydroxypropylmethyl cellulose, ethyl cellulose, starch, dextrin, dextran, pectin, and pullulan, and derivatives thereof.

25. A medicinal oral preparation for treating colitis comprising a core containing 0.5 to 10 wt % of budesonide and, covering the core, an inner layer containing one or more cationic polymers and an outer layer containing one or more anionic polymers.

26. The medicinal oral preparation for treating colitis according to Claim 25, wherein the preparation is designed so that, in a disintegration test comprising vertical movement for 2 hours in a first solution of pH 1.2, subsequent vertical movement for 2 hours in a second solution of pH 7.4, and final vertical movement in a third solution of pH 6.4, the average disintegration initiation time and the average disintegration completion time each fall within a period from 35 min to 130 min after starting the vertical movement in the third solution.

27. The medicinal oral preparation for treating colitis according to Claim 26, wherein the preparation is designed so that the average disintegration initiation time is 35 min to 115 min and the average disintegration completion time is 50 min to 130 min after starting the vertical movement in the third solution.

28. The medicinal oral preparation for treating colitis according to any one of Claims 25 to 27, wherein the core contains one or more types selected from the group consisting of a binder, a disintegrating agent, and a saccharide.

29. The medicinal oral preparation for treating colitis according to Claim 28, wherein the core contains 5 to 40 wt % of the binder, and the mixing ratio of budesonide and the binder is 1:10 to 1:30.

30. The medicinal oral preparation for treating colitis according to either Claim 28 or 29, wherein the binder is one or more types selected from the group consisting of crystalline cellulose, gum arabic, sodium alginate, ethyl cellulose, agar, a carboxyvinyl polymer, carmellose, gelatin, low-substituted hydroxypropyl cellulose, starch, dextrin, hydroxypropyl cellulose, hydroxypropylmethyl cellulose, pectin, polyvinyl pyrrolidone, Macrogol, and methyl cellulose.

31. The medicinal oral preparation for treating colitis according to Claim 28, wherein the core contains 2 to 15 wt % of the disintegrating agent, and the mixing ratio of budesonide and the disintegrating agent is 1:2 to 1:10.

32. The medicinal oral preparation for treating colitis according to either Claim 28 or 31, wherein the disintegrating agent is one or more types selected from the group consisting of crospovidone, pregelatinized starch, sodium carboxymethyl starch, carmellose, calcium carmellose, sodium carmellose, powdered agar, sodium croscarmellose, low-substituted hydroxypropyl cellulose, starch, dextrin, hydroxyethylmethyl cellulose, hydroxypropyl starch, Macrogol, and mannitol.

33. The medicinal oral preparation for treating colitis according to Claim 32, wherein the disintegrating agents are crospovidone and low-substituted hydroxypropyl cellulose.

34. The medicinal oral preparation for treating colitis according to Claim 33, wherein the mixing ratio of crospovidone and low-substituted hydroxypropyl cellulose is 1:2.5 to 1:10.

35. The medicinal oral preparation for treating colitis according to Claim 28, wherein the core contains 40 to 80 wt % of a saccharide.

36. The medicinal oral preparation for treating colitis according to either Claim 28 or 35, wherein the saccharide is one or more types selected from the group consisting of monosaccharides and disaccharides of lactose, fructose, sucrose, glucose, xylitol, maltose, mannitol, and sorbitol, polysaccharides of cellulose, hydroxypropyl cellulose, hydroxypropylmethyl cellulose, ethyl cellulose, starch, dextrin, dextran, pectin, and pullulan, and derivatives thereof.
